# EUROPÄISCHE PATENTSCHRIFT

(11) **EP 3 456 362 B1**
(45) Veröffentlichungstag und Bekanntmachung des Hinweises auf die Patenterteilung: **05.05.2021**
(21) Anmeldenummer: 18203318.3
(22) Anmeldetag: 17.10.2017
(51) Int. Cl.: A61L 24/04, A61L 24/06, A61L 27/16, A61L 27/50

(54) **KNOCHENZEMENT-KIT ZUR VERWENDUNG IN EINEM VERFAHREN ZUR HERSTELLUNG EINES POLYMETHYLMETHACRYLAT-KNOCHENZEMENTS**
BONE CEMENT KIT FOR USE IN A METHOD FOR PRODUCING A POLYMETHYL METHACRYLATE BONE CEMENT
ENSEMBLE DE CIMENT OSSEUX DESTINÉ À ÊTRE UTILISÉ DANS UN PROCÉDÉ DE FABRICATION D'UN CIMENT OSSEUX DE POLYMÉTHACRYLATE DE MÉTHYLE

(30) Priorität: 11.11.2016 DE 102016222158
(43) Veröffentlichungstag der Anmeldung: 20.03.2019
(62) Teilanmeldung aus: 17196738.3
(73) Patentinhaber: Heraeus Medical GmbH, 61273 Wehrheim (DE)
(72) Erfinder: VOGT, Sebastian, 99092 Erfurt (DE); KLUGE, Thomas, 56179 Vallendar (DE)
(74) Vertreter: Heraeus IP

(56) Entgegenhaltungen:
- EP-A1- 2 402 041
- EP-A2- 2 687 239
- DE-A1-102014 109 234

## Beschreibung

Gegenstand der Erfindung ist ein Knochenzement-Kit.

Polymethylmethacrylat-(PMMA)-Knochenzemente gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs, bis dieser erstarrt.

Polymethylmethacrylat-Knochenzemente können in geeigneten Mischbechern mit Hilfe von Spateln durch manuelles Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind:
US 6,033,105 A, US 5,624,184 A, US 4,671,263 A, US 4,973,168 A, US 5,100,241 A,
WO 99/67015 A1, EP 1 020 167 A2, US 5,586,821 A, EP 1 016 452 A2, DE 36 40 279 A1,
WO 94/26403 A1, EP 1 005 901 A2, US 5,344,232 A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepack-Mischsysteme wurden in den Patenten EP 0 692 229 A1, DE 10 2009 031178 B3, US 5,997,544 A, WO 00/35506 A1 und US 5,588,745 A beschrieben.

Bei der Verwendung fast aller bisher bekannten Full-Prepack-Mischsysteme muss der medizinische Anwender die Zementkomponenten, das Zementpulver und die Monomerflüssigkeit manuell durch Betätigung von Mischvorrichtungen, wie Mischstäben mit Rührflügeln, zu einem Zementteig vermischen. Die Homogenität des gebildeten Zementteigs hängt wesentlich von der Durchführung der manuellen Vermischung ab und kann daher Schwankungen unterliegen.

Mischverfahren und Einrichtungen, die kein manuellen Mischen der Komponenten erfordern, wie sie in WO 00/35506 A1 und US 5,588,745 A beschrieben sind, erfordern kompliziert aufgebaute Einrichtungen und/oder funktionieren häufig nur unter speziellen Voraussetzungen.

So wurde in WO 00/35506 A1 zur Vermeidung von möglichen Inhomogenitäten des Zementteigs eine Lagerungs- und Mischvorrichtung vorgeschlagen, bei der die Zementkomponenten ohne manuell angetriebene mechanische Vermischung miteinander vermischt werden. Das Polymethylmethacrylat-Knochenzementpulver wird dabei in einer Kartusche gelagert, wobei das Zementpulver das gesamte Volumen der Kartusche ausfüllt und die Zwischenräume zwischen den Partikeln des Zementpulvers ein solches Volumen haben, das dem Volumen der Monomerflüssigkeit entspricht, die zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird und durch Vakuum, das an einem Vakuumanschluss an der Unterseite der Kartusche anliegt, durch das Zementpulver gezogen wird, wobei die in den Zwischenräumen der Zementpartikel befindliche Luft durch die Monomerflüssigkeit verdrängt wird. Auf eine mechanische Durchmischung des gebildeten Zementteigs mit einem Rührer wird dabei verzichtet. Nachteilig an diesem System ist, dass alle bisher auf dem Markt verfügbaren Zementpulver mit dieser Vorrichtung nicht angemischt werden können, weil die schnell anquellenden Zementpulverpartikel nach einem Eindringen der Monomerflüssigkeit in das Zementpulver in eine Tiefe von ungefähr 1-2 cm eine gelartige Barriere bilden und die weitere Migration der Monomerflüssigkeit durch das gesamte Zementpulver behindern. Konventionelle Zementpulver zeigen zudem das Phänomen, dass auf Grund der unterschiedlichen Oberflächenenergien die Pulverpartikel durch Methylmethacrylat nur schlecht benetzt werden. Dadurch dringt das Methylmethacrylat nur relativ langsam in das Zementpulver ein.

Die DE 10 2014 109 234 A1 beschreibt ein Zwei-Komponenten-Prothesenmaterial enthaltend eine flüssige Monomerkomponente und eine pulverförmige Komponente. Die pulverförmige Komponente kann anorganische Füllstoffe, wie beispielsweise pyrogenes Siliziumdioxid, enthalten

Die EP 2 687 239 A1 beschreibt ein Kit zur Herstellung eines Knochenzements aus zwei Pasten A und B, wobei eine oder beide Pasten einen schwer- oder unlöslichen Füllstoff, wie beispielsweise pyrogenes Siliziumdioxid, enthalten kann.

Die EP 2 402 041 A1 Knochenzement-Kit-Zusammensetzung, wobei die Zusammensetzung große (Meth)acrylat-Polymer-Partikel mit einem Durchmesser von 10 bis 60 µm und kleine (Meth)acrylat-Polymer-Partikel mit einem Durchmesser von 0,1 bis 2 µm umfasst und der Anteil der kleinen (Meth)acrylat-Polymer-Partikel in einem Bereich von 5 bis 30 Gew.-% bezogen auf alle (Meth)acrylat-Polymer-Partikel liegt.

Die Aufgabe der Erfindung besteht somit in der Entwicklung eines Knochenzement-Kit, welches zum Anmischen keine komplizierten Vorrichtungen benötigt und aus welchem unabhängig von der Bedienperson reproduzierbar und schnell ein applizierbarer Knochenzement bereitstellbar ist.

Die Aufgabe wurde durch ein erfindungsgemäßes Knochenzement-Kit gelöst. Das Knochenzement-Kit umfasst a) ein Zementpulver, das mindestens ein partikuläres Polymethylmethacrylat oder Polymethylmethacrylat-Copolymer der Siebfraktion kleiner 100 µm, einen Initiator, und mindestens ein in Methylmethacrylat unlöslichen, partikulären oder faserförmigen Additiv, wobei das Additiv ein pyrogenes Siliziumdioxid ist und ein Aufsaugvermögen größer gleich 9,4 g Methylmethacrylat pro Gramm Additiv bei Raumtemperatur besitzt, und b) eine Monomerlösung, die mindestens ein Methylmethacrylat und einen Aktivator enthält.

Es wurde überraschend gefunden, dass es möglich ist, durch einfaches In-Kontakt-Bringen eines im folgenden definierten Zementpulvers mit einer nachstehend definierten Monomerflüssigkeit einen klebfreien, plastisch verformbaren Knochenzementteig herzustellen, der selbstständig durch radikalische Polymerisation aushärtet, ohne dass es notwendig ist, den Zementteig manuell oder mit Hilfe von technischen Hilfsmitteln zu vermischen.

Die Erfindung beruht auf der überraschenden Beobachtung, dass durch Zusatz eines in Methylmethacrylat unlöslichen, partikulären oder faserförmigen Additivs in Form eines pyrogenen Siliziumdioxids, das ein Aufsaugvermögen von größer 9,4 g Methylmethacrylat pro Gramm Additiv bei Raumtemperatur besitzt, zu einem Zementpulver eines niedrig-viskosen Zements, ein modifiziertes Zementpulver erhalten wird, in das Monomerflüssigkeit über eine Strecke von mindestens 5 cm eingepresst werden kann. Das Additiv verbessert überraschend auch die Benetzung des Zementpulvers mit Monomerflüssigkeit. Es erleichtert das Eindringen der Monomerflüssigkeit in das Zementpulver. Das Additiv hat dabei einen "Docht-Effekt". Das Additiv leitet schon in sehr geringen Mengen ab 0,1 Gew.-% die Monomerflüssigkeit in das Innere des Zementpulvers. Weiterhin verzögert das Additiv das Verkleben der Polymerpartikel, wodurch die Ausbildung einer blockierenden Gelschicht verzögert wird und das Eindringen der Monomerflüssigkeit in das Zementpulver begünstigt wird.

Weiterhin wurde überraschend gefunden, dass durch den Zusatz des Additivs zu einem Zementpulver auch durch Vakuumeinwirkung Monomerflüssigkeit über eine Strecke von mindestens 3,0 cm ausgehend vom Einleitungspunkt der Monomerflüssigkeit in einer Vorrichtung gemäß WO 00/35506 A1 gesaugt werden kann. Es wurde weiterhin gefunden, dass durch Einpressen von Monomerflüssigkeit in das erfindungsgemäße Zementpulver ein homogener Zementteig entsteht, so dass eine mechanische Vermischung der Zementkomponenten nicht notwendig ist. Auf diese Weise entsteht nach Vermischung des Zementpulvers mit der Monomerflüssigkeit sofort ein klebfreier, plastisch verformbarer Zementteig, der verarbeitbar und applizierbar ist und selbstständig durch radikalische Polymerisation aushärtet.

Bevorzugt werden Monomerflüssigkeit und Zementpulver unmittelbar vor und während der Absorption so angeordnet, dass die Monomerflüssigkeit in Bezug auf die Erdoberfläche unterhalb des Zementpulvers angeordnet ist. Dadurch können im Zementpulver enthaltene Lufteinschlüsse auf einfache Weise entweichen.

Die Ausdrücke "enthalten" und "umfassen", wie sie hier in der vorliegenden Erfindung verwendet werden, schließen auch "bestehen aus" und "im Wesentlichen bestehen aus" mit ein.

Unter Raumtemperatur wird erfindungsgemäß eine Temperatur von 23°C verstanden.

Die Monomerflüssigkeit umfasst Methylmethacrylat als Monomer.

Geeignete Polymere in dem Zementpulver sind Polymethylmethacrylat und Copolymere von Methylmethacrylat mit einem oder mehreren damit copolymerisierbaren Monomeren, wie Methylacrylat, Styren und Ethylacrylat, welche als Pulver vorliegen.

Das Polymerpulver ist ein Polymerpulver der Siebfraktion < 100 µm. Damit enthält das Polymer ausschließlich Partikel, die durch ein Sieb mit einer Maschenweite von 100 µm fallen.

Es kann vorgesehen sind, dass die Zwischenräume im Zementpulver zwischen 25 Volumenprozent und 40 Volumenprozent liegen.

Der Gewichtsanteil des Polymers der Siebfraktion < 100 µm kann in breiten Bereichen variieren, wobei der Gewichtsanteil in dem Zementpulver bevorzugt im Bereich von 69,5-99,3 liegt.

Das Zementpulver enthält außerdem einen Polymerisationsinitiator. Bei dem Polymerisationsinitiator handelt es sich vorzugsweise um einen aktivierbaren Polymerisationsinitiator, z.B. ein Peroxid.

Peroxide sind bevorzugt. Das Peroxid weist vorzugsweise keine freien aciden Gruppen auf. Bei dem Peroxid kann es sich um ein organisches Peroxid oder ein anorganisches Peroxid handeln, wie z.B. Dialkylperoxide oder Hydroperoxide. Beispielsweise kann das Peroxid aus der Gruppe ausgewählt sein, die aus Dibenzoylperoxid, Cumol-hydroperoxid, 1,1,3,3-Tetramethylbutylperoxid, t-Butylperoxid, t-Amyl-hydroperoxid, Di-Isopropylbenzen-monohydroperoxid und einer Mischung aus mindestens zwei davon besteht. Gemäß einer besonders bevorzugten Ausführungsform ist das Peroxid aus der Gruppe ausgewählt, die aus Dibenzoylperoxid und Dilauroylperoxid besteht. Besonders bevorzugt ist mit Wasser phlegmatisiertes Dibenzoylperoxid mit einem Wassergehalt von weniger als 30 Gew.-%, bevorzugt weniger als 28 Gew.-%.

Der Initiator wird dem Zementpulver in einem Anteil von 0,4-3,0 Gew.-%, bezogen auf das Zementpulver zugegeben.

Das Zementpulver enthält ferner ein in Methylmethacrylat unlösliches, partikuläres oder faserförmiges Additiv, wobei das Additiv ein pyrogenes Siliziumdioxid ist und ein Aufsaugvermögen größer gleich 9,4 g Methylmethacrylat pro Gramm Additiv bei Raumtemperatur besitzt,
Zur Bestimmung des Aufsaugvermögens der Additive wurde die aus der Pharmazie bekannte Enslin-Apparatur (C.-D. Herzfeldt, J. Kreuter (Hrsg.): Grundlagen der Arzneiformenlehre. Galenik 2, Springer Verlag Berlin Heidelberg New York, 1999, S. 79-80.) vereinfacht. Es wurde ein Glasfiltertiegel 1D3 der Firma Schott verwendet. Es wurde zuerst die Taramasse des Glasfiltertiegels bestimmt. Dann wurden jeweils 3,000 g bzw. 1,000 g des Additivs in den Glasfiltertiegel eingewogen. Der Glasfiltertiegel wurde auf eine Saugflasche aufgesetzt. Zum Additiv wurden 20 ml Methylmethacrylat gegeben, so dass das Additiv vollständig bedeckt war. Das nicht vom Additiv aufgesaugte Methylmethacrylat lief durch den Glasfiltertiegel nach unten ab. Nach 15 Minuten wurde der Glasfiltertiegel mit dem Additiv und dem aufgesaugten Methylmethacrylat ausgewogen und die Masse des aufgesaugten Methylmethacrylats bestimmt. Die Bestimmung wurde jeweils dreimal wiederholt und der Mittelwert bestimmt. Als Referenz wurde der Glasfiltertiegel mit Methylmethacrylat ohne zugesetztes Additiv in gleicher Weise behandelt.

Vorteilhaft ist es, wenn das Additiv kovalent gebundene Hydroxylgruppen an seiner Oberfläche besitzt. Besonders vorteilhaft sind dabei besonders Si-OH-Gruppen und alkoholische OH-Gruppen. Durch die oberflächlich angeordneten OH-Gruppen hat das Additiv eine hohe Oberflächenenergie, wodurch eine gute Benetzbarkeit des Additivs mit Methylmethacrylat gegeben ist.

Die pyrogenen Kieselsäuren Aerosil® 380 und Aerosil® 300 sind besonders geeignet. Daneben ist es auch möglich, durch Sol-Gel-Prozesse erzeugtes Siliziumdioxid als Additiv zu verwenden.

Bevorzugt hat das Additiv eine Partikelgröße der Siebfraktion kleiner 100 µm, bevorzugt der Siebfraktion kleiner 50 µm und ganz besonders bevorzugt der Siebfraktion kleiner 10 µm.

Dem Knochenzementpulver kann wenigstens ein Röntgenopaker zugemischt werden. Beim Röntgenopaker kann es sich um einen fachüblichen Röntgenopaker handeln, bevorzugt in partikulärer Form. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen, wie Calciumcarbonat und Calciumsulfat besteht. Dabei sind Zirkoniumdioxid, Bariumsulfat, Calciumcarbonat und Calciumsulfat bevorzugt. Diese Röntgenopaker weisen vorzugsweise einen mittleren Teilchendurchmesser im Bereich von 10 nm bis 500 µm auf. Die Konzentration an zugemischtem Röntgenopaker, insbesondere die Konzentration an Zirkoniumdioxid, in dem Knochenzementpulver liegt bevorzugt in einem Bereich von 0 bis 30 Gew.-%, besonders bevorzugt in einem Bereich von 0 bis 15,0 Gew.-%.

In dem trockenen Zementpulver lassen sich pharmazeutische Wirkstoffe, wie Antiphlogistika, Bisphosphonate, Wachstumsfaktoren und bevorzugt Antiinfektiva und/oder Antiseptika relativ problemlos über einen längeren Zeitraum lagern. Als Antiinfektiva sind insbesondere Gentamicin, Tobramycin, Clindamycin, Vancomycin, Fosfomycin, Colistin und Daptomycin bevorzugt, die in Form von in Wasser leicht löslichen Salzen oder auch in Form von in Wasser gering löslichen Salzen oder Komplexen eingesetzt werden können. Als Antiseptika sind Octenidin, Dequaliniumchlorid, Polyhexanid, Calciumperoxid, und Benzalkoniumchlorid bevorzugt.

Die Monomerflüssigkeit enthält einen Aktivator. Aktivatoren aus der Gruppe der aromatischen Amine sind bevorzugt. Als Aktivator sind N,N-Di-methyl-p-toluidin, N,N-Di-methyl-o-toluidin, N,N-Dimethyl-anilin, N,N-Bis-hydroxyethyl-p-toluidin besonders bevorzugt.

Die Monomerflüssigkeit enthält bevorzugt auch einen radikalischen Stabilisator aus der Gruppe der Chinone oder der sterisch gehinderten Phenole. Als radikalischer Stabilisator werden p-Hydrochinon, o-Hydrochinon, 2,6-Di-t-butyl-p-hydrochinon, 2-t-butyl-p-hydrochinon und 2,6-Di-t-butyl-4-methyl-phenol bevorzugt.

Die Monomerflüssigkeit kann ferner eine farbgebende Substanz enthalten. Bei der farbgebenden Substanz handelt es sich z.B. um Farbpigmente, Lebensmittelfarbstoffe oder Farblacke. Beispiele sind E101, E104, E132, E141 (Chlorophyllin), E142, Riboflavin, Lissamingrün und Farblack Grün, das Aluminiumsalz einer Mischung aus E104 und E132. Chlorophyllin E141 ist bevorzugt.

Bevorzugt sind die Antiinfektiva und Antiseptika in dem Zementpulver in einem Anteil von 1,0 bis 10 Gew.-% enthalten.

In einer ersten Ausführungsform ist das Zementpulver aus 0,0-15,0 Gew.-% Röntgenopaker, 0,4-3,0 Gew.-% Dibenzoylperoxid, 79,5-99,3 Gew.-% Polymethylmethacrylat und/oder Polymethylmethacrylat-Copolymer, 0,1-2,5 Gew.-% Additiv zusammengesetzt.

In einer zweiten Ausführungsform ist das Zementpulver aus 1,0-10 Gew.-% eines Antiinfektivums oder Antiseptikums, 0,0-15,0 Gew.-% Röntgenopaker, 0,4-3,0 Gew.-% Dibenzoylperoxid, 69,5-98,3 Gew.-% Polymethylmethacrylat und/oder Polymethylmethacrylat-Copolymer, 0,1-2,5 Gew.-% Additiv zusammengesetzt.

Das erfindungsgemäße Knochenzement-Kit enthält das oben beschriebene Zementpulver und die oben beschriebene Monomerflüssigkeit, die Methylmethacrylat und mindestens einen wie oben beschriebenen Aktivator.

Zementpulver und Monomerflüssigkeit stellen gesonderte Komponenten dar und können im nicht ausgehärteten Zustand separat gelagert werden.

Die Erfindung wird durch nachstehende Beispiele näher erläutert, ohne dass diese jedoch die Erfindung beschränken.

### Beispiel 1: Bestimmung des Aufsaugvermögens des Additivs

Für die Bestimmung des Aufsaugvermögens des Additivs wurden folgende Ausgangsstoffe verwendet:
Methylmethacrylat (Sigma-Aldrich)
Stärke (Sigma-Aldrich, Siebfraktion <100 µm)
Cellulose (Sigma-Aldrich, Siebfraktion <100 µm)
Aerosil® 380 (Evonik, Partikelgröße ∼ 7 nm)

Für die Bestimmung des Aufsaugvermögens der Additive Stärke, Cellulose und Aerosil®380 wurde ein Glasfiltertiegel 1D3 der Firma Schott Mainz verwendet. Es wurde zuerst die Taramasse des Glasfiltertiegels bestimmt. Dann wurden jeweils 3,000 g Additiv und beim Aerosil® 380 1,000g in den Glasfiltertiegel eingewogen. Der Glasfiltertiegel mit dem eingewogenen Additiv wurde auf eine Saugflasche aufgesetzt. Zum Additiv wurden 10 ml Methylmethacrylat gegeben, so dass das Additiv vollständig bedeckt war. Das nicht vom Additiv aufgesaugte Methylmethacrylat lief durch den Glasfiltertiegel nach unten ab. Nach 15 Minuten wurde der Glasfiltertiegel mit dem Additiv und dem aufgesaugten Methylmethacrylat ausgewogen und die Masse des aufgesaugten Methylmethacrylats bestimmt. Die Bestimmung wurde jeweils dreimal wiederholt und der Mittelwert ermittelt. Als Referenz wurde der Glasfiltertiefgel mit Methylmethacrylat ohne zugesetztes Additiv in gleicher Weise behandelt.

| Additiv | Aufsaugvermögen [g Methylmethacrylat/g Additiv] |
|---|---|
| Stärke (Vergleichsbeispiel) | 0,7 |
| Cellulose (Vergleichsbeispiel) | 1,8 |
| Aerosil® 380 | 9,4 |

### Beispiel 2: Mischen von Zementpulver und Monomerflüssigkeit

Es wurden folgende Ausgangsstoffe für die Herstellung der nachfolgenden Zementpulver 1 bis 11 verwendet:
Methylmethacrylat-methylacrylat-copolymer,
75%iges Dibenzoylperoxid (BPO, phlegmatisiert mit 25 Gew.-% Wasser)
Zirkoniumdioxid,
Stärke (Sigma-Aldrich, Siebfraktion <100 µm)
Cellulose (Sigma-Aldrich, Siebfraktion <100 µm)
Aerosil® 380 (Evonik, Partikelgröße ∼ 7 nm)

Die Komponenten der Zementpulver wurden in 1000 ml Plastikflaschen eingewogen. Danach erfolgte eine Homogenisierung der Zementpulver durch Mischen mit einem TURBULA®-Mischer (Willy A. Bachofen AG). Die Mischzeit betrug 30 Minuten.

Zusammensetzung der Zementpulver 1-3 (Vergleichsbeispiele)

| Zementpulver-Nr. | PMMA-co-MA | ZrO₂ | 75%iges BPO | Stärke [g] |
|---|---|---|---|---|
| | [g] | [g] | [g] | |
| 1 | 32,47 | 6,00 | 0,53 | 1,00 |
| 2 | 31,47 | 6,00 | 0,53 | 2,00 |
| 3 | 30,47 | 6,00 | 0,53 | 3,00 |

Zusammensetzung der Zementpulver 4-6 (Vergleichsbeispiele)

| Zementpulver-Nr. | PMMA-co-MA | ZrO₂ | 75%iges BPO | Cellulose [g] |
|---|---|---|---|---|
| | [g] | [g] | [g] | |
| 4 | 32,47 | 6,00 | 0,53 | 1,00 |
| 5 | 31,47 | 6,00 | 0,53 | 2,00 |
| 6 | 30,47 | 6,00 | 0,53 | 3,00 |

Zusammensetzung der Zementpulver 7-11

| Zementpulver-Nr. | PMMA-co-MA | ZrO₂ | 75%iges BPO | Aerosil® 380 [g] |
|---|---|---|---|---|
| | [g] | [g] | [g] | |
| 7 | 33,43 | 6,00 | 0,53 | 0,04 |
| 8 | 33,34 | 6,00 | 0,53 | 0,12 |
| 9 | 33,22 | 6,00 | 0,53 | 0,25 |
| 10 | 32,97 | 6,00 | 0,53 | 0,50 |
| 11 | 32,72 | 6,00 | 0,53 | 0,75 |

Eine transparente, hohlzylinderförmige Kunststoffkartusche mit einem Innendurchmesser von 35 mm wurde mit einem gasdurchlässigen, aber für Zementpulverpartikel undurchlässigen Verschlusskopf für die folgenden Versuche eingesetzt. In die Kartusche wurde 40,00 g Zementpulver gefüllt. Danach wurde der Hohlraum mit dem Zementpulver durch einen für Zementpulverpartikel undurchlässigen, aber für Gase und Flüssigkeiten durchlässigen, axial im Kunststoffrohr verschiebbaren ersten Kolben verschlossen. In den Hohlraum hinter dem ersten Kolben wurde eine Palacos-Ampulle mit 20 ml Monomerflüssigkeit eingelegt. Anschließend wurde ein für Gase, Flüssigkeiten und Zementpulverpartikel undurchlässiger, axial in der Kunststoffkartusche verschiebbarer zweiter Kolben eingesetzt. Sodann wurde das Kunststoffrohr senkrecht gestellt, mit dem Verschlusskopf nach oben. Mit Hilfe einer handbetriebenen, mechanischen Auspressvorrichtung (Palamixgun) wurde dann der zweite Kolben in Richtung des Verschlusskopfs gedrückt. Zuerst brach die Ampulle und die Monomerflüssigkeit und die Splitter wurden in Richtung des zweiten Kolbens gepresst. Die Monomerflüssigkeit wurde dabei durch den zweiten Kolben in das Zementpulver gepresst. Dabei wurde das Zementpulver komplett mit der aufsteigenden Monomerflüssigkeit über eine Strecke von 5,8 cm benetzt. Nach einer Wartezeit von wenigen Sekunden wurde der Verschlusskopf geöffnet und der gebildete Zementteig heraus gepresst.

In einer Variante des Versuchs wurde am Verschlusskopf ein Vakkuumanschluss angesetzt. Es wurden jeweils 40,00 g Zementpulver in die Kartusche gefüllt. Die Kartusche wurde anschließend mit einem für Gase und Flüssigkeiten durchlässigen Kolben verschlossen. Die Kartusche wurde mit dem Kopf nach unten gelagert und Vakuum wurde angelegt. Dann wurden jeweils 20 ml Monomerflüssigkeit auf den für Gase und Flüssigkeiten durchlässigen Kolben gegeben und Vakuum angelegt. Bei allen Zementen wurde die Monomerflüssigkeit über eine Strecke von ca. 4,0 cm durch das Zementpulver gesaugt.

### Beispiel 3: Prüfung nach ISO5833

Für die Herstellung der nachfolgenden Zementpulver wurden folgende Ausgangsstoffe verwendet:
Methylmethacrylat-methylacrylat-copolymer,
75%iges Dibenzoylperoxid (BPO, phlegmatisiert mit 25 Gew.-% Wasser)
Zirkoniumdioxid,
Gentamicinsulfat,
Clindamycinhydrochlorid,
Vancomycinhydrochlorid,
Daptomycin,
Trometamol-Fosfomycin,
Octenidindihydrochlorid
Stärke (Sigma-Aldrich, Siebfraktion <100 µm)
Cellulose (Sigma-Aldrich, Siebfraktion <100 µm)
Aerosil® 380 (Evonik, Partikelgröße ∼ 7 nm)

Als Referenzmaterial wurde der PMMA-Knochenzement PALACOS® LV+G (Lot. 7732, exp. 2017-06) verwendet.

Die Komponenten der Zementpulver wurden in 1000 ml Plastikflaschen eingewogen. Danach erfolgte eine Homogenisierung der Zementpulver durch Mischen mit einem TURBULA-Mischer (Willy A. Bachofen AG). Die Mischzeit betrug 30 Minuten.

Zusammensetzung der Zementpulver 12-14 (Vergleichsbeispiele)

| Zementpulver-Nr. | PMMA-co-MA | ZrO₂ | 75%iges BPO | Stärke [g] |
|---|---|---|---|---|
| | [g] | [g] | [g] | |
| 12 | 32,47 | 6,00 | 0,53 | 1,00 |
| 13 | 31,47 | 6,00 | 0,53 | 2,00 |
| 14 | 30,47 | 6,00 | 0,53 | 3,00 |

Zusammensetzung der Zementpulver 15-17 (Vergleichsbeispiele)

| Zementpulver-Nr. | PMMA-co-MA | ZrO₂ | 75%iges BPO | Cellulose [g] |
|---|---|---|---|---|
| | [g] | [g] | [g] | |
| 15 | 32,47 | 6,00 | 0,53 | 1,00 |
| 16 | 31,47 | 6,00 | 0,53 | 2,00 |
| 17 | 30,47 | 6,00 | 0,53 | 3,00 |

Zusammensetzung der Zementpulver 18-22

| Zementpulver-Nr. | PMMA-co-MA | ZrO₂ | 75%iges BPO | Aerosil® 380 [g] |
|---|---|---|---|---|
| | [g] | [g] | [g] | |
| 18 | 33,43 | 6,00 | 0,53 | 0,04 |
| 19 | 33,34 | 6,00 | 0,53 | 0,12 |
| 20 | 33,22 | 6,00 | 0,53 | 0,25 |
| 21 | 32,97 | 6,00 | 0,53 | 0,50 |
| 22 | 32,72 | 6,00 | 0,53 | 0,75 |

Zusammensetzung der Zementpulver 23-28

| Zementpulver-Nr. | Wi rkstoff | PMMA-co-MA [g] | ZrO₂ [g] | 75%iges BPO [g] | Aerosil® 380 [g] |
|---|---|---|---|---|---|
| 23 | 1,00 g Gentamicinsulfat | 33,22 | 6,00 | 0,53 | 0,25 |
| 24 | 1,00 g Clindamycinhydrochlorid | 33,22 | 6,00 | 0,53 | 0,25 |
| 25 | 1,00 g Vancomycin-hydrochlorid | 33,22 | 6,00 | 0,53 | 0,25 |
| 26 | 1,00 g Daptomycin | 33,22 | 6,00 | 0,53 | 0,25 |
| 27 | 1,00 g Trometamol-Fosfomycin | 33,22 | 6,00 | 0,53 | 0,25 |
| 28 | 1,00 g Octenidinhydro-chlorid | 33,22 | 6,00 | 0,53 | 0,25 |

Für die Herstellung von Zementteig und der Fertigung von Prüfkörpern wurden 10 ml PALACOS®-Monomerflüssigkeitsampullen (Lot. 5276, exp. 2010-10) der Firma Heraeus Medical GmbH eingesetzt. Die Monomerflüssigkeit einer 10 ml Monomerampulle besteht aus 9,20 g Methylmethacrylat, 0,19 g N,N-Dimethyl-p-toluidin, 10 ppm p-Hydrochinon und 0,2 mg Chlorophyllin (E141).

Es wurden mit den Zementpulvern 12-28 sowie dem Zementpulver Palacos LV+G durch Vermischung mit der Monomerflüssigkeit Zementteigmuster hergestellt. Bei den Zementpulvern 12-22 wurden dazu jeweils 20,0 g Zementpulver mit 10 ml Monomerflüssigkeit vermischt und bei den Zementpulvern 23-28 wurden 20,5 g Zementpulver mit 10 ml Monomerflüssigkeit homogenisiert. Aus dem jeweils gebildeten Zementteig wurden streifenförmige Prüfkörper mit den Abmessungen (75 mm x 10 mm x 3,3 mm) für die Bestimmung der Biegefestigkeit und des Biegemoduls hergestellt und zylindrische Formkörper (Durchmesser 6 mm, Höhe 12 mm) für die Bestimmung der Druckfestigkeit angefertigt. Die Probekörper wurden dann 24 Stunden bei 23 ± 1 °C an der Luft gelagert. Danach wurden die 4-Punkt-Biegefestigkeit, das Biegemodul und die Druckfestigkeit der Prüfkörper mit einer Zwick-Universalprüfmaschine ermittelt.

Ergebnisse der Prüfung der Biegefestigkeit, des Biegemoduls und der Druckfestigkeit gemäß der ISO 5833 der Zementproben 12-28

| Zementpulver-Nr. | 4-Punkt-Biegefestigkeit [MPa] | Biegemodul [MPa] | Druckfestigkeit [MPa] |
|---|---|---|---|
| 12 | 63,9 ± 1,3 | 2783 ± 87 | 93,0 ± 1,6 |
| 13 | 63,4 ± 1,1 | 2799 ± 80 | 93,7 ± 1,3 |
| 14 | 59,9 ± 1,4 | 2747 ± 103 | 91,6 ± 1,3 |
| 15 | 69,8 ± 1,3 | 2977 ± 96 | 94,4 ± 1,2 |
| 16 | 68,3 ± 2,0 | 2995 ± 135 | 94,4 ± 0,9 |
| 17 | 65,8 ± 1,1 | 2954 ± 57 | 92,5 ± 1,0 |
| 18 | 73,8 ± 1,3 | 3066 ± 59 | 95,6 ± 0,9 |
| 19 | 68,1 ± 2,6 | 2877 ± 20 | 97,7 ± 2,2 |
| 20 | 73,5 ± 1,4 | 2919 ± 78 | 93,2 ± 3,2 |
| 21 | 66,0 ± 2,8 | 2870 ± 60 | 84,5 ± 3,8 |
| 22 | 61,3 ± 3,2 | 2823 ± 3,1 | 89,3 ± 3,6 |
| 23 | 62,2 ± 1,9 | 2871 ± 129 | 94,8 ± 1,4 |
| 24 | 62,5 ± 2,5 | 2788 ± 138 | 92,6 ± 2,2 |
| 25 | 65,8 ± 3,7 | 3004 ± 55 | 94,6 ± 2,6 |
| 26 | 67,5 ± 1,4 | 2970 ± 24 | 93,6 ± 2,0 |
| 27 | 63,8 ± 2,4 | 2881 ± 75 | 91,2 ± 1,0 |
| 28 | 55,6 ± 1,4 | 2829 ± 91 | 89,8 ± 1,9 |
| Referenz Palacos LV+G | 65,2 ± 1,7 | 2851 ± 93 | 91,2 0,9 |

Die ISO5833 schreibt für ausgehärteten Polymethylmethacrylat-Knochenzement eine Biegefestigkeit von mindestens >50 MPa, ein Biegemodul von mindestens >1800 MPa und eine Druckfestigkeit von mindestens >70 MPa vor. Die Ergebnisse der Bestimmung der Biegefestigkeit, des Biegemoduls und der Druckfestigkeit der mit den Zementpulvern 12- 28 hergestellten Zementmuster zeigen, dass die Zementmuster den Anforderungen der ISO5833 hinsichtlich der Biegefestigkeit, des Biegemoduls und der Druckfestigkeit genügen und diese deutlich übertreffen.

## Patentansprüche

1. Knochenzement-Kit, **gekennzeichnet durch**
a) ein Zementpulvers, enthaltend
- mindestens ein partikuläres Polymethylmethacrylat oder Polymethylmethacrylat-Copolymer der Siebfraktion kleiner 100 µm,
- einen Initiator, und
- mindestens ein in Methylmethacrylat unlösliches, partikuläres oder faserförmiges Additiv, wobei das Additiv ein pyrogenes Siliziumdioxid ist und ein Aufsaugvermögen größer gleich 9,4 g Methylmethacrylat pro Gramm Additiv bei Raumtemperatur besitzt,
wobei das Additiv im Zementpulver in einer Menge von 0,1 bis 2,5 Gew.-% bezogen auf das Gesamtgewicht des Zementpulvers enthalten ist,
b) eine Monomerlösung, enthaltend
- mindestens ein Methylmethacrylat und
- einen Aktivator.

2. Kit nach Anspruch 1, **dadurch gekennzeichnet,**
**dass** das Additiv kovalent gebundene Hydroxylgruppen an seiner Oberfläche besitzt.

3. Kit nach einem der Ansprüche 1 bis 2, **dadurch gekennzeichnet, dass** das Additiv eine Partikelgröße der Siebfraktion kleiner 100 µm, bevorzugt der Siebfraktion kleiner 50 µm und ganz besonders bevorzugt von der Siebfraktion kleiner 10 µm hat.

4. Kit nach einem der Ansprüche 1 bis 3, **dadurch gekennzeichnet, dass** das Polymerpulver Dibenzoylperoxid als Initiator enthält.

5. Kit nach einem der Ansprüche 1 bis 4, **dadurch gekennzeichnet, dass** die Monomerflüssigkeit mindestens einen Aktivator aus der Gruppe der aromatischen Amine enthält.

6. Kit nach einem der Ansprüche 1 bis 5, **dadurch gekennzeichnet, dass** die Monomerflüssigkeit mindestens einen radikalischen Stabilisator aus der Gruppe der Chinone oder der sterisch gehinderten Phenole enthält.

7. Kit nach einem der Ansprüche 1 bis 6, **dadurch gekennzeichnet, dass** das Zementpulver einen partikulären Röntgenopaker enthält.

8. Kit nach einem der Ansprüche 1 bis 7, **dadurch gekennzeichnet, dass** das Zementpulver
0,0-15,0 Gew.-% Röntgenopaker,
0,4-3,0 Gew.-% Dibenzoylperoxid,
79,5-99,3 Gew.-% Polymethylmethacrylat und/oder Polymethylmethacrylat-Copolymer, und
0,1-2,5 Gew.-% Additiv enthält.

9. Kit nach einem der Ansprüche 1 bis 8, **dadurch gekennzeichnet, dass** das Zementpulver
1,0-10 Gew.-% Antiinfektivum oder Antiseptikum,
0,0-15,0 Gew.-% Röntgenopaker,
0,4-3,0 Gew.-% Dibenzoylperoxid,
69,5-98,3 Gew.-% Polymethylmethacrylat und/oder Polymethylmethacrylat-Copolymer, und
0,1-2,5 Gew.-% Additiv enthält.

## Claims

1. A bone cement kit, **characterized by**
a) a cement powder, containing
- at least one particulate polymethyl methacrylate or polymethyl methacrylate copolymer of the sieve fraction of less than 100 µm,
- an initiator, and
- at least one particulate or fibrous additive, which is insoluble in methyl methacrylate,
wherein the additive is a pyrogenic silica and has an absorption capacity of greater than or equal to 9.4 g of methyl methacrylate per gram of additive at room temperature,
wherein the additive is contained in the cement powder in a quantity of 0.1 to 2.5% by weight, based on the total weight of the cement powder,
b) a monomer solution, containing
- at least one methyl methacrylate and
- an activator.

2. The kit according to claim 1, **characterized in**
**that** the additive has covalently bound hydroxyl groups on its surface.

3. The kit according to any one of claims 1 to 2, **characterized in that** the additive has a particle size of the sieve fraction of less than 100 µm, preferably of the sieve fraction of less than 50 µm, and particularly preferably of the sieve fraction of less than 10 µm.

4. The kit according to any one of claims 1 to 3, **characterized in that** the polymer powder contains dibenzoyl peroxide as initiator.

5. The kit according to any one of claims 1 to 4, **characterized in that** the monomer liquid contains at least one activator from the group of the aromatic amines.

6. The kit according to any one of claims 1 to 5, **characterized in that** the monomer liquid contains at least one radical stabilizer from the group of the quinones or of the sterically hindered phenols.

7. The kit according to any one of claims 1 to 6, **characterized in that** the cement powder contains a particulate radiopaque.

8. The kit according to any one of claims 1 to 7, **characterized in that** the cement powder contains
0.0-15.0% by weight of radiopaque,
0.4-3.0% by weight of dibenzoyl peroxide,
79.5-99.3% by weight of polymethyl methacrylate and/or polymethyl methacrylate copolymer, and
0.1-2.5% by weight of additive.

9. The kit according to any one of claims 1 to 8, **characterized in that** the cement powder contains
1.0-10% by weight of anti-infective or antiseptic,
0.0-15.0% by weight of radiopaque,
0.4-3.0% by weight of dibenzoyl peroxide,
69.5-98.3% by weight of polymethyl methacrylate and/or polymethyl methacrylate copolymer, and
0.1-2.5% by weight of additive.

## Revendications

1. Kit de ciment osseux, **caractérisé par**
a) une poudre de ciment contenant
- au moins un polyméthacrylate de méthyle ou copolymère de polyméthacrylate de méthyle particulaire d'une fraction granulométrique inférieure à 100 µm,
- un initiateur et
- au moins un additif insoluble dans le méthacrylate de méthyle, particulaire ou fibreux, l'additif étant un dioxyde de silicium pyrogène et possédant à température ambiante une capacité d'absorption supérieure ou égale à 9,4 g de méthacrylate de méthyle par gramme d'additif,
l'additif étant contenu dans la poudre de ciment dans une quantité de 0,1 à 2,5% en poids par rapport au poids total de la poudre de ciment,
b) une solution de monomère contenant
- au moins un méthacrylate de méthyle et
- un activateur.

2. Kit conformément à la revendication 1, **caractérisé en ce que** l'additif possède à sa surface des groupes hydroxyles liés par covalence.

3. Kit conformément à l'une des revendications 1 à 2, **caractérisé en ce que** l'additif a une taille de particule d'une fraction granulométrique inférieure à 100 µm, préférablement d'une fraction granulométrique inférieure à 50 µm et encore plus préférablement d'une fraction granulométrique inférieure à 10 µm.

4. Kit conformément à l'une des revendications 1 à 3, **caractérisé en ce que** la poudre de polymère contient du peroxyde de dibenzoyle comme initiateur.

5. Kit conformément à l'une des revendications 1 à 4, **caractérisé en ce que** le liquide de monomère contient au moins un activateur du groupe des amines aromatiques.

6. Kit conformément à l'une des revendications 1 à 5, **caractérisé en ce que** le liquide de monomère contient au moins un stabilisateur radicalaire du groupe des quinones ou des phénols stériquement entravés.

7. Kit conformément à l'une des revendications 1 à 6, **caractérisé en ce que** la poudre de ciment contient un agent radio-opaque particulaire.

8. Kit conformément à l'une des revendications 1 à 7, **caractérisé en ce que** la poudre de ciment contient
de 0,0 à 15,0% en poids d'agent radio-opaque,
de 0,4 à 3,0% en poids de peroxyde de dibenzoyle,
de 79,5 à 99,3% en poids de polyméthacrylate de méthyle et/ou copolymère de polyméthacrylate de méthyle et
de 0,1 à 2,5% en poids d'additif.

9. Kit conformément à l'une des revendications 1 à 8, **caractérisé en ce que** la poudre de ciment contient
de 1,0 à 10% en poids d'anti-infectieux ou d'antiseptique,
de 0,0 à 15,0% d'agent radio-opaque,
de 0,4 à 3,0% en poids de peroxyde de dibenzoyle,
de 69,5 à 98,3% en poids de polyméthacrylate de méthyle et/ou copolymère de polyméthacrylate de méthyle et
de 0,1 à 2,5% en poids d'additif.
